# EUROPEAN PATENT APPLICATION

(11) **EP 4 745 851 A1**
(43) Date of publication of application: **20.05.2026**
(21) Application number: 25215368.9
(22) Date of filing: 12.11.2025
(51) Int. Cl.: G06N 20/20, G16H 50/70

(54) **SYSTEMS AND METHODS FOR IMPROVING ACCURACY OF A PRIMARY PREDICTIVE MODEL BASED ON A RESIDUAL MODEL**

(30) Priority: 13.11.2024 US 202418946805
(71) Applicant: Medidata Solutions, Inc., New York, NY 10014 (US)
(72) Inventor: BLUM, Kevin, New York, 10014 (US); OKIDI, Evon, Boston, 02110 (US); YANG, Eric, New York, 10014 (US)
(74) Representative: D Young & Co LLP

(57) **Abstract**

Relates to improving accuracy of a primary predictive model based on a residual model. A method includes determining, using a primary predictive model, a first set of training prediction residuals based on a first labeled training set and a first set of testing prediction residuals based on a first labeled testing set. A second dataset includes a second labeled training set, labeled based on the first set of training prediction residuals, and a second labeled testing set, labeled based on the first set of testing prediction residuals. The method further includes training the residual model using the second labeled training set. The primary predictive model is adjusted based on a set of training predictions and a set of testing predictions of the residual model to produce more accurate predictions.

## Description

### FIELD

The present disclosure generally relates to improving accuracy of a primary predictive model based on a residual model.

### BACKGROUND

Collaborative modeling while preserving data privacy among collaborators presents significant challenges, particularly in scenarios where two parties wish to collaborate on a predictive model but cannot pool their data into a single modeling dataset due to privacy concerns or other constraints.

Federated learning aims to address the problem of data governance and privacy by enabling collaborative training of algorithms without exchanging the data itself. In federated learning, a central aggregator coordinates multiple collaborators on a machine learning task, preserving data privacy for each participant. This approach is especially relevant in the life sciences domain, where health data is sensitive and tightly regulated. In a typical federated learning approach, each user trains the model locally on their own data and then uploads the model to a shared server for centralized aggregation. The shared server updates the model with data from all collaborators, allowing each party to access an improved model trained on a broader dataset.

However, conventional federated learning approaches have several drawbacks. These methods require alignment of datasets across collaborators, verification of feature definitions, and checking feature distributions. They also necessitate alignment of the shared modeling algorithm, such as an artificial neural network architecture. Additionally, a third party or host is needed for the centralized server to maintain data privacy, which introduces the risk of privacy leaks due to adversarial attacks. Attributing improvements in model performance is challenging, and the algorithms suitable for federated learning, which generally depend on updating weighted parameters, are typically limited to artificial neural networks.

### SUMMARY

Particular aspects are set out in the appended independent claims. Various optional embodiments are set out in the dependent claims.

In view of the deficiencies in conventional approaches discussed above, it is imperative to provide a technical solution to the technical problem of ensuring predictive model accuracy by introducing methodologies and tools that leverage residuals and interactive adjustments from secondary entities to enhance model performance, while maintaining data privacy and security.

Disclosed embodiments provide a method for improving predictive model accuracy. The method includes defining a prediction task; creating a dataset, including training and testing sets; generating initial predictions and calculating residuals; providing the residuals to a secondary entity; generaZting additional predictions based on the residuals; and adjusting the initial predictions using the additional predictions to enhance accuracy.

Disclosed embodiments provide a method for enhancing predictive model performance. The method includes defining a modeling cohort and prediction task; creating a modeling dataset, including training and testing sets, and labeling training samples; making initial predictions and calculating prediction residuals; and providing the prediction residuals to a collaborator; creating an additional dataset and aligning it with the initial dataset; training a model on the prediction residuals and generating additional predictions; and adjusting the initial model using the additional predictions to improve model accuracy.

Disclosed embodiments provide a method for improving predictive modeling accuracy. The method includes defining a modeling cohort and a prediction task by a primary modeling team; engineering a modeling dataset, determining training and testing sets, and labeling the training samples by the primary modeling team; generating initial predictions and calculating prediction residuals using the dataset by the primary modeling team; providing the training and testing set labels and corresponding prediction residuals to a collaborator team; creating a modeling dataset, engineering features, and aligning the train and test split with those of the primary modeling team by the collaborator team; training a model on the prediction residuals and generating additional predictions using the trained model by the collaborator team, and providing the additional predictions to the primary modeling team; and adjusting the initial model using the additional predictions to create an adjusted model with enhanced accuracy.

Disclosed embodiments provide a method for enhancing predictive modeling accuracy. The method includes defining, by a primary modeling team, a modeling cohort and a prediction task; engineering, by the primary modeling team, a modeling dataset, determining training and testing sets, and labeling the training samples; using, by the primary modeling team, the dataset to make initial predictions and calculating prediction errors, referred to as residuals; delivering, by the primary modeling team, the training and testing set labels and the corresponding prediction residuals to a collaborator team; creating, by the collaborator modeling team, a modeling dataset from a cohort that aligns with the defined modeling cohort and prediction task, engineering features, and aligning the train and test split with those of the primary modeling team; training, by the collaborator modeling team, a model on the prediction residuals received from the primary modeling team and using the trained residual model to make predictions on the test set, and delivering the resulting predictions back to the primary modeling team; and adjusting, by the primary modeling team, the initial model using the predictions received from the collaborator modeling team, resulting in an adjusted model that demonstrates improved accuracy over the initial model, and evaluating the degree of improvement obtained from the residual model predictions.

In one aspect, the disclosed embodiments provide methods, systems, and computer-readable media for improving accuracy of a primary predictive model based on a residual model. The method includes: determining, using a primary predictive model, a first set of training prediction residuals based on a first labeled training set and a first set of testing prediction residuals based on a first labeled testing set; producing a second dataset for a residual model, the second dataset comprising a second labeled training set, labeled based on the first set of training prediction residuals, and a second labeled testing set, labeled based on the first set of testing prediction residuals; training the residual model using the second labeled training set; determining, using the residual model, a set of training predictions based on the second labeled training set and a set of testing predictions based on the second labeled testing set; and adjusting the primary predictive model based on the set of training predictions and the set of testing predictions of the residual model to produce more accurate predictions.

Embodiments one or more of the following features, alone or in combination.

The method may include producing a first dataset for the primary predictive model, the first dataset comprising the first labeled training set and the first labeled testing set; and training the primary predictive model using the first labeled training set. The producing of the first dataset for the primary predictive model may be based on a first set of historical patient-level data and the producing of the second dataset for the residual model may be based on a second set of historical patient-level data, the first set of patient-level data being different from the second set of patient-level data.

The producing of the second dataset for the residual model may include receiving values and labels of the first set of training prediction residuals and values and labels of the first set of testing prediction residuals, without receiving data from the first set of historical patient-level data. The producing of the first dataset for the primary predictive model may include defining a modeling cohort and a prediction task. The prediction task may include predicting clinical trial enrollment. The prediction task may include predicting functional disability of a patient. The determining of the first set of training prediction residuals and the first set of testing prediction residuals may include: performing predictions based on the first labeled training set and the first labeled testing set; and comparing the predictions to labels of the first labeled training set and the first labeled testing set to compute respective residuals thereof.

The adjusting of the primary predictive model may include subtracting the set of training predictions and the set of testing predictions of the residual model from predictions of the primary predictive model, the predictions of the primary predictive model being based on the first labeled training set and the first labeled testing set. The adjusting of the primary predictive model may include retuning hyperparameters of the primary predictive model to cause a subsequent set of training prediction residuals and a subsequent set of testing prediction residuals to be closer to the first set of training labels and the first set of testing labels.

### BRIEF DESCRIPTION OF THE DRAWINGS

Figure 1 is a diagram of a system for improving accuracy of a predictive model, according to disclosed embodiments.
Figure 2 is a diagram of the primary model of the system of Fig. 1.
Figure 3 is a diagram of the residual model of the system of Fig. 1.
Figure 4 is a flowchart of a method for improving accuracy of a predictive model, in accordance with disclosed embodiments.
Figures 5-12 are scatter plots of selected features used in a Full Model, and their Shapley Additive Explanations (SHAP) values, from an example of the disclosed approaches applied to a publicly-available dataset for a cohort of patients.
Figures 13-15 are scatter plots of selected features in a primary model in the example and their SHAP values.
Figures 16-20 are scatter plots of selected features in a residual model in the example and their SHAP values.
Figure 21 is a table of feature importance rank, based on SHAP values for the Full Model, primary model, and residual model of the example.
Figure 22 is a table showing the test set error for the primary model, the Adjusted Model, produced in accordance with the approaches described herein, and the Full Model of the example.

Where considered appropriate, reference numerals may be repeated among the drawings to indicate corresponding or analogous elements. Moreover, some of the blocks depicted in the drawings may be combined into a single function.

### DETAILED DESCRIPTION

In the following detailed description, numerous specific details are set forth in order to provide a thorough understanding of embodiments of the presently taught approaches. However, it will be understood by those of ordinary skill in the art that the embodiments of the presently taught approaches may be practiced without these specific details. In other instances, well-known methods, procedures, components, and circuits have not been described in detail so as not to obscure the presently taught approaches.

As discussed above, conventional approaches to collaborative modeling may require setup through a centralized server of a trusted third party, extensive data exchanges, and data anonymization. These require time to setup and implement, whereas the disclosed approaches require no central server and have only limited datapoints exchanged. For example, in federated learning techniques, model weights are exchanged through a shared server and are, therefore, vulnerable to potential attacks, whereas the disclosed approaches do not rely on such exchanges. Furthermore, the disclosed approaches result in a reduction of computing needs, because conventional federated learning techniques require a network of devices and generally depend on artificial neural network algorithms, which increase both computing costs and complexity.

There is a significant independence of modeling tasks in the disclosed approaches, because the modeling task is split between the primary and residual models. Therefore, each collaborator is free to make modeling choices tailored to their own dataset, thereby improving performance of the model as a whole. For example, rather than jointly updating model weights, as in conventional federated learning techniques, feature importance can be generated for both the primary and residual models independently. Also, the disclosed approaches provide transparency with respect to improvements in model performance, so both collaborators can have a representation of the prediction error and can therefore independently quantify the lift or improvement resulting from the residual model.

Among the advantages of the disclosed approaches is that they allow both teams in a collaborative arrangement to maintain their data independently without the need for data sharing. Each team may have its own modeling features, thereby addressing concerns related to the sensitivity of proprietary data and contractual limitations on data granularity. Ideally, both teams would pool their data together to build a unified model. However, due to various restrictions, they are unable to do so. Thus, a benefit of the disclosed approaches is that they effectively provide joint modeling without sharing of sensitive data. It should be noted that while the underlying motivation for ensuring data privacy and security may stem from ethical, legal, and regulatory concerns, the actual process of achieving and maintaining data privacy and security is a technical endeavor, as described in detail herein.

In some alternative solutions to the approaches described herein, feature deliveries to primary modelers (or "clients") are anonymized to mask any data points derived from a single source, but this reduces the predictive signal. The data exchange process is relatively complex, which tends to create a barrier to client adoption. In some cases, extensive feature definitions are provided and a file is delivered with dataset joining keys and, e.g., approximately 110 features. A detailed walkthrough may be provided to assist the client in integrating the features with the client's own modeling dataset. The disclosed approaches, in contrast, involve the delivery of joining keys, which the client provides, and the error (e.g., residuals) of the client's model. The disclosed approaches do not require any actual values (i.e., data points) from the client, thereby preserving the privacy of the client's dataset.

Furthermore, such alternative solutions depend on the quality of the client's predictive model and their data science team's modeling choices. For example, a highly effective set of predictive features might be provided, but poor modeling choices by the client could undermine their effectiveness. The disclosed approaches decouple the use of such features from the client's modeling choices, ensuring more robust predictive performance and a fairer assessment of the collaborator's contribution to the client's prediction accuracy, in the sense that both the client and the collaborator can see how much the residual model is improving the primary model predictions.

Although the disclosed embodiments relate to client modeling support, it extends to any scenario in which two parties seek to collaborate on a predictive model but cannot pool their data into a single modeling dataset due to privacy concerns or other reasons.

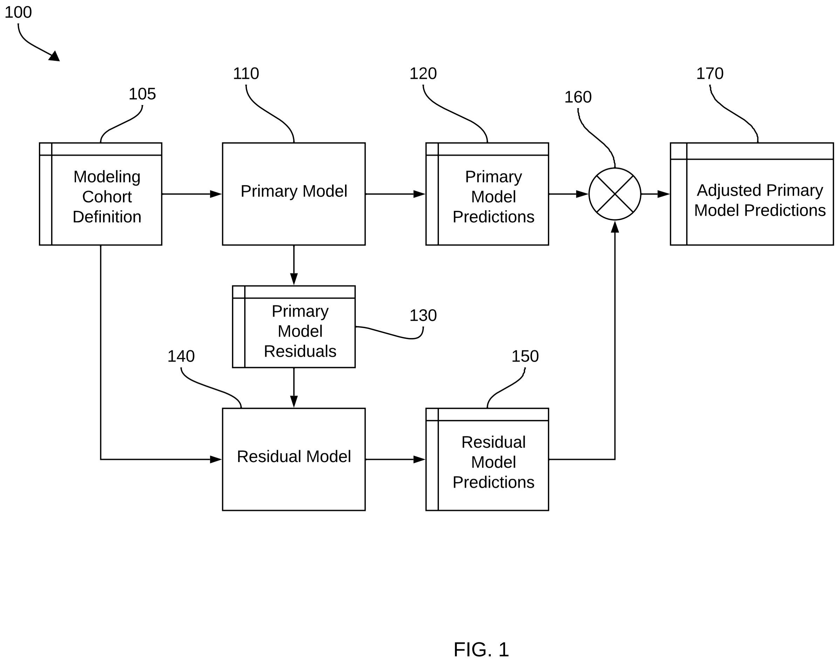

Figure 1 is a diagram of a system 100 for improving accuracy of a predictive model. The system 100 implements a predictive modeling method that, in disclosed embodiments, involves both a primary predictive model, e.g., primary model 110, and a residual predictive model, e.g., residual model 140, to enhance prediction accuracy. In implementations, this may involve a collaborative predictive modeling approach that involves both a primary modeling team and a collaborator modeling team.

The primary model 110 uses statistical, machine learning, and/or other computational techniques to generate predictions based on input data. In embodiments, a gradient boosting machine (GBM) may be used for the primary model 110 due to the ability of such models to handle complex patterns and interactions and to provide high predictive accuracy. A GBM may provide boosting techniques in which models are sequentially built to correct the errors of previous models. Various other types of machine learning tools, e.g., neural networks, may be used to implement the primary model 110.

A modeling cohort definition 105 is input to the primary model 110 and the residual model 140. The modeling cohort definition 105 is a collection of data points or instances that define the scope and characteristics of the data to be modeled. The modeling cohort definition 105 serves as the dataset for subsequent modeling processes. As discussed in further detail below, the system 100 may implement methods which include defining a modeling cohort and prediction task and creating a modeling dataset, including training and testing sets, and labeling training samples.

In a typical scenario, a modeling team (or "primary modeling team") has a specific goal, such as modeling clinical trial enrollment at the study site level. The team defines the cohort they want to use, such as breast cancer trials. Their objective might be, for example, to identify the best recruitment sites for an upcoming breast cancer trial. The primary modeling team may engineer a modeling dataset, including determining the training and testing sets and labeling the training samples accordingly. The primary modeling team undertakes the model training process, e.g., for the primary model 110, using both training and validation (or testing) data. In some cases, there may be a team dedicated to designing and testing the algorithm, with a focus on predicting the desired outcome. This may involve feature engineering, data processing, building the prediction model, and evaluating its performance.

In the example depicted, the primary model 110 produces two outputs: primary model predictions 120 and primary model residuals 130 (i.e., primary model prediction residuals). The primary model predictions 120 represent the initial forecast or estimation generated by the primary model 110 for each data point within the modeling cohort. The primary model residuals 130 are the differences between the actual observed values and the primary model predictions 120. These residuals provide an indication of the prediction errors or deviations of the primary model 110.

In the example depicted, the primary model residuals 130 are input to the residual model 140, which is a predictive model (or algorithm) that is specifically trained to model the residuals produced by the primary model. The residual model 140 produces residual model predictions 150 based on the input residuals. These predictions are, in effect, used to correct or adjust the predictions made by the primary model 110. In implementations, the primary modeling team initiate a data transfer in which the training and testing set labels, along with the corresponding prediction residuals for those samples, are sent to a system (or model) implemented by a collaborator modeling team. This transfer of data allows the collaborator team to further refine the prediction process. Such data could be transferred in a particular format, e.g., CSV or JSON, or stored in cloud storage for retrieval by the collaborator team.

In the example depicted, the residual model predictions 150 are subtracted from the primary model predictions 120 in an adjustment process 160. The subtraction yields the adjusted primary model predictions 170, which represent the enhanced and more accurate forecast or estimation for each data point in the modeling cohort. These adjusted predictions, in effect, incorporate the insights from both the primary model 110 and the residual model 140. Thus, the system 100 effectively leverages the complementary strengths of the primary model 110 and the residual model 140 to achieve a more accurate and reliable predictive performance. By addressing the residual errors of the primary model 110 through a dedicated residual model 140, the system 100 effectively ensures that relationships between the primary model labels and the residual model dataset features are accounted for, thereby providing more accurate predictions.

In some implementations of the system 100, a primary modeling team may define a prediction task and a primary dataset and arrange for a collaborator modeling team to implement a residual model. In such a case, each team would maintain the privacy and security of their own data. The primary model would be fit on the primary dataset and the prediction error (i.e., the residuals) would be delivered to the collaborator team. The collaborator team would fit a model on those residuals, using the collaborator's own dataset, features, and other parameters, and deliver the predictions back to the primary team as the residual predictions. The primary modeling team would apply the residual model's predictions as an adjustment to their original prediction, thereby obtaining an overall improvement in model accuracy while preserving data privacy for both parties.

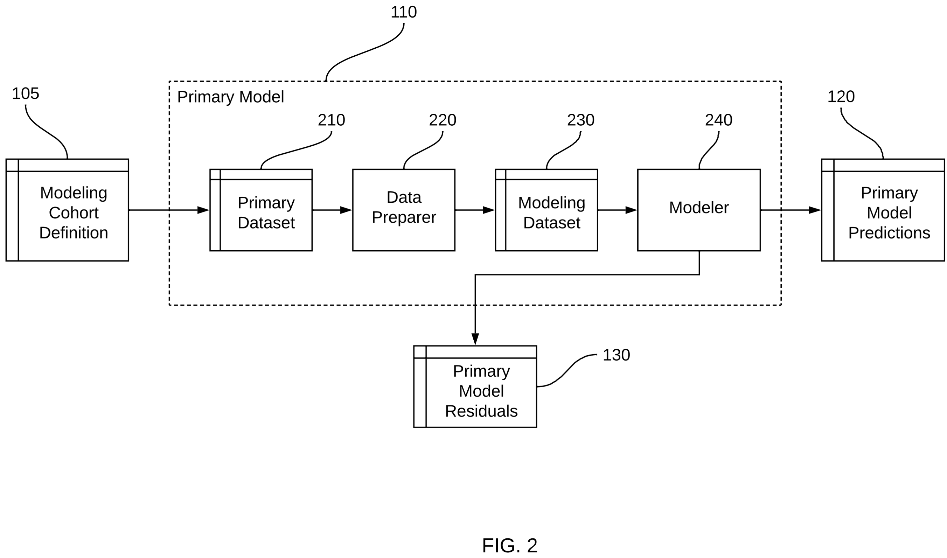

Figure 2 is a diagram of the primary model 110 of the system 100 of Fig. 1, showing the detailed functioning of the primary model 110 within a predictive modeling system. The primary model 110 is responsible for generating initial predictions and calculating residuals based on the modeling cohort definition 105 (see also, Fig. 1). As discussed above, the modeling cohort definition 105 specifies the scope and characteristics of the data to be modeled, thereby forming the basis for subsequent data processing and modeling. For the sake of simplicity and to facilitate the description thereof, the primary model 110 is delineated by dashed lines which surround various components. However, it is to be understood that these components are not necessarily part of the primary model 110 per se, but, rather, may be entirely independent components and may have entirely separate implementations.

A primary dataset 210 is produced based on the modeling cohort definition 105. The primary dataset 210 comprises raw data points or instances that fall within the defined cohort and serves as the initial collection of data to be processed and analyzed. Although the primary dataset 210 is depicted as being within the dashed lines delineating the primary model 110, it is not necessarily a component thereof, as explained above.

The primary dataset 210 is input to a data preparer 220, which, in implementations, may be a module or component responsible for preprocessing and feature engineering. Although the data preparer 220 is depicted as being within the dashed lines delineating the primary model 110, it is not necessarily a component thereof, as explained above. The preprocessing may include, for example, data cleaning, normalization, transformation, and handling of missing values to ensure data quality and consistency. The feature engineering may include, for example, the selection and/or creation of features (or variables) to better represent the underlying patterns in the data.

The data preparer 220 outputs a modeling dataset 230, which is structured and refined for effective modeling. The modeling dataset 230 may include, for example, a number of observations, i.e., data instances or samples, a number of features, i.e., independent variables or attributes that describe each observation, and a number of dependent variables, i.e., the target variables or outcomes that the model aims to predict. Although the modeling dataset 230 is depicted as being within the dashed lines delineating the primary model 110, it is not necessarily a component thereof, as explained above.

The modeling dataset 230 is input to a modeler 240, which is responsible for facilitating exploratory data analysis (EDA) and the building of the primary model 110. EDA involves statistical and graphical analysis to understand the data distribution, identify patterns, and detect anomalies. The building (or modeling) of the primary model 110 involves selecting and training a predictive algorithm using the modeling dataset 230. The algorithm could be a statistical model, machine learning model, and/or other computational techniques.

The modeler 240 outputs two key results: the primary model predictions 120, and the primary model residuals 130 (see also, Fig. 1). As discussed above, the primary model predictions 120 are the initial predictions generated by the primary model 110 for each observation in the modeling dataset 230. They represent the model's forecast or estimation of the dependent variables. The primary model residuals 130 (i.e., primary model prediction residuals) are the differences between the actual observed values of the dependent variables and the primary model predictions 120. The residuals indicate the prediction errors or deviations of the primary model.

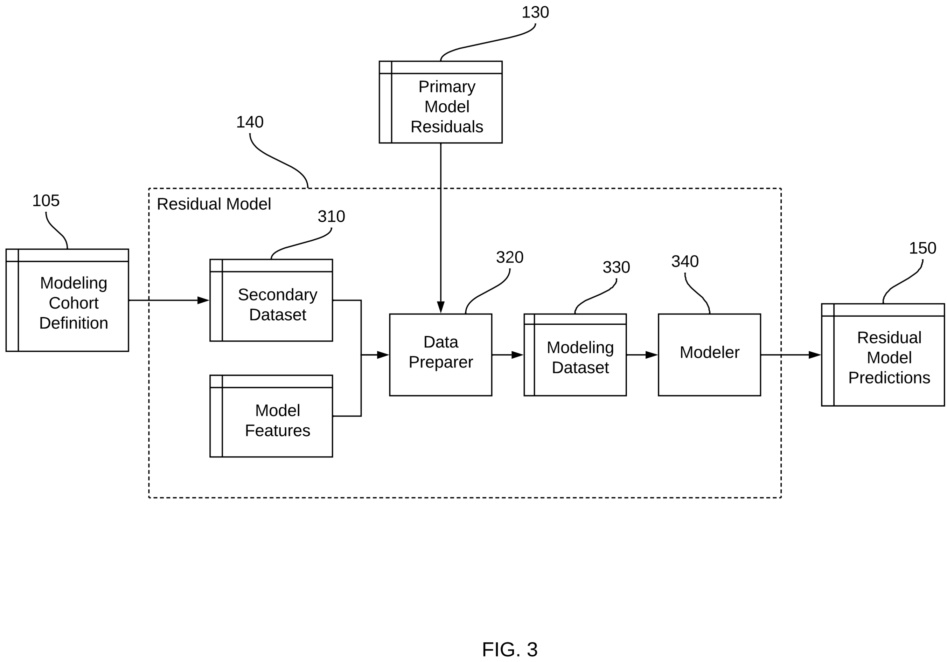

Figure 3 is a diagram of the residual model 140 of the system 100 of Fig. 1, showing the detailed functioning of the residual model 140 within a predictive modeling system. The residual model 140 is responsible for refining and correcting the initial predictions made by the primary model 110 based on based on the modeling cohort definition 105 and the primary model residuals 130 (see also, Fig. 1). As discussed above, the modeling cohort definition 105 specifies the scope and characteristics of the data to be modeled, thereby forming the basis for subsequent data processing and modeling. For the sake of simplicity and to facilitate the description thereof, the residual model 140 is delineated by dashed lines which surround various components. However, it is to be understood that these components are not necessarily part of the residual model 140 per se, but, rather, may be entirely independent components and may have entirely separate implementations.

A secondary dataset 310 is produced based on the modeling cohort definition 105. The secondary dataset 310 comprises raw data points or instances that fall within the defined cohort and serves as the initial collection of data to be processed and analyzed. Although the secondary dataset 310 is depicted as being within the dashed lines delineating the residual model 140, it is not necessarily a component thereof, as explained above.

In implementations, the collaborator modeling team may generate a modeling dataset from a cohort definition that aligns with the modeling cohort definition 105 generated by the primary modeling team. The modeling cohort definition 105 is depicted with the same reference number in the primary model 110 and the residual model 140, but it may be independently generated by the collaborator team based on specifications known to both teams rather than being the result of a data transfer. The collaborator modeling team may create a cohort definition and independently engineer a feature set. The collaborator team typically aligns the training and testing split of the secondary dataset 310 to match the split used for the primary dataset 210. This helps to ensure consistency and compatibility between the datasets used by both teams.

The secondary dataset 310 is input to a data preparer 320, which, in implementations, may be a module or component responsible for preprocessing and feature engineering. Although the data preparer 320 is depicted as being within the dashed lines delineating the residual model 140, it is not necessarily a component thereof, as explained above. The preprocessing may include, for example, data cleaning, normalization, transformation, and handling of missing values to ensure data quality and consistency. The feature engineering may include, for example, the selection and/or creation of features (or variables) to better represent the underlying patterns in the data.

The data preparer 320 outputs a modeling dataset 330, which is structured and refined for effective modeling. The modeling dataset 330 may include, for example, data instances or samples, features (or independent variables or attributes) that describe each observation, and dependent variables, which are the target variables or outcomes that the model aims to predict. In embodiments, the modeling dataset 330 may include, for example: data instances or samples and historical metrics or variables that describe each observation and which are pertinent for modeling the residuals. Although the modeling dataset 330 is depicted as being within the dashed lines delineating the residual model 140, it is not necessarily a component thereof, as explained above.

The modeling dataset 330 is input to a modeler 340, which is responsible for facilitating exploratory data analysis (EDA) and the building of the residual model 140. EDA involves statistical and graphical analysis to understand the data distribution, identify patterns, and detect anomalies. The building of the residual model 140 (or "modeling") involves selecting and training a predictive algorithm using the modeling dataset 330. The predictive algorithm is specifically designed to model the residuals from the primary model (primary model residuals 130), effectively capturing patterns or information that the primary model 110 may have missed. The predictive algorithm could be a statistical model, machine learning model, and/or other computational techniques.

The modeler 340 outputs the residual model predictions 150 (see also, Fig. 1). These predictions represent the adjustments or corrections to be applied to the initial predictions of the primary model 110 (primary model predictions 120). They are derived from the analysis of, e.g., the historical metrics and primary model residuals 130 by the residual model 140. As explained above, the residual model predictions 150 are subtracted from the primary model predictions 120 in an adjustment process 160 (see Fig. 1). The subtraction yields the adjusted primary model predictions 170, which represent the enhanced and more accurate forecast or estimation for each data point in the modeling cohort. These adjusted predictions, in effect, incorporate the insights from both the primary model 110 and the residual model 140.

In implementations, the collaborator modeling team initiates a data transfer in which the residual model predictions 150 are sent to the system implemented by the primary modeling team, which integrates them with the primary model predictions 120. As discussed above, this involves subtracting the residual model predictions from the primary model predictions to produce adjusted primary model predictions. Thus, the adjusted model is more accurate than the primary model alone, as it benefits from the refinements provided by the residual model. The primary modeling team can also evaluate the degree of model improvement obtained from incorporating the residual model predictions.

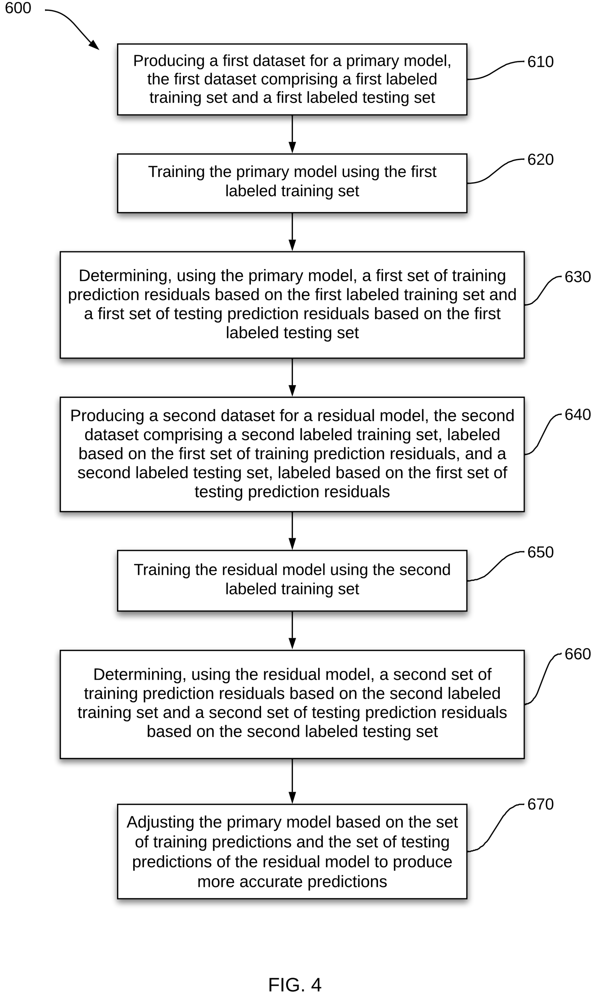

Figure 4 is a flowchart of a method 600 for improving accuracy of a predictive model, in accordance with disclosed embodiments. The method 600 includes producing a first dataset for a primary model, the first dataset comprising a first labeled training set and a first labeled testing set (610). The method further includes training the primary model using the first labeled training set (620). The method further includes determining, using the primary model, a first set of training prediction residuals based on the first labeled training set and a first set of testing prediction residuals based on the first labeled testing set (630). The method further includes producing a second dataset for a residual model, the second dataset comprising a second labeled training set, labeled based on the first set of training prediction residuals, and a second labeled testing set, labeled based on the first set of testing prediction residuals (640). The method further includes training the residual model using the second labeled training set

(650). The method further includes determining, using the residual model, a set of training predictions based on the second labeled training set and a set of testing predictions based on the second labeled testing set (660). The method further includes adjusting the primary model based on the set of training predictions and the set of testing predictions of the residual model to produce more accurate predictions (670).

In embodiments, the adjusting of the primary model (670) may involve subtracting the set of training predictions and the set of testing predictions of the residual model from predictions of the primary predictive model, the predictions of the primary predictive model being based on the first labeled training set and the first labeled testing set.

The adjusting of the primary model (670) may also involve adjustments (or changes) to the primary model itself. For example, the adjusting may involve retuning hyperparameters of the primary predictive model to cause a subsequent set of training prediction residuals and a subsequent set of testing prediction residuals to be closer to the first set of training labels and the first set of testing labels.

In implementations, the primary modeling team may evaluate the overall performance of the adjusted model (i.e., the primary model with its predictions adjusted based on the predictions received from the residual model) to provide feedback on the performance of the adjusted model to the residual modeling team (or collaborator team). In some cases, based on the feedback, the collaborator team may provide insights into areas where the primary model could be improved or where additional features might be beneficial. For example, it may be suggested that the primary modeling team perform feature re-engineering, which may include adding new features and/or transforming existing ones to better capture the patterns in the adjusted predictions. This may further involve model selection and tuning, e.g., trying different models and/or retuning hyperparameters to improve performance. This may further include data augmentation, which may involve incorporating additional data sources and/or more samples to enhance the training process.

The need to improve the accuracy of predictive models is a technical problem because it requires the development, implementation, and maintenance of complex methodologies and tools. Predictive modeling is a cornerstone in various technological fields, including healthcare, finance, marketing, and beyond. The accuracy of such models is crucial for making reliable forecasts that inform decision-making processes. However, achieving high accuracy is challenging due to the inherent limitations of the data available to any individual predictive model. Traditional predictive models often fall short because they cannot incorporate additional relevant insights that may be available to other predictive models. This limitation leads to suboptimal model performance and inaccurate predictions, which can result in significant inefficiencies and errors in practical applications.

The disclosed approaches provide a technical solution to this technical problem by enhancing the accuracy of predictive models through the use of residuals and interactive adjustments. The disclosed approaches are designed to systematically improve model performance by incorporating additional data from a second, independent predictive model.

Specifically, the technical solution involves generating predictions and calculating residuals, which represent the prediction errors, using a particular "primary" predictive model. The residuals and relevant metadata are used in an independent residual (or secondary) predictive model, which may use data and features not available to the primary model. The collaborator uses the residuals to train a residual model, thereby leveraging the unique features of the residual model to generate additional predictions. This introduces new insights that were not initially available to the primary model. The predictions produced by the residual model are used to adjust the predictions of the primary model, which results in an adjusted primary model with improved accuracy. This interactive adjustment effectively reduces the prediction errors of the primary model.

A number of technical benefits and advantages arise from application of the disclosed approaches. As discussed above, by leveraging residuals and additional insights from a residual model, the disclosed approaches significantly improve the accuracy of predictive models. This technical improvement is critical for applications where precision is paramount, such as predicting metrics relating to conducting clinical trials and their effectiveness and success.

The disclosed approaches enable interaction between independently-implemented predictive models without the need for raw data sharing, thereby helping to maintain data privacy and security. This aspect is particularly important in sectors like healthcare, where patient data confidentiality is crucial. While the underlying motivation for ensuring data privacy and security may stem from ethical, legal, and regulatory concerns, the actual process of achieving and maintaining data privacy and security is a technical endeavor.

The disclosed approaches provide scalability and flexibility, in that, for example, they are versatile and can be applied to various prediction tasks across different domains. The disclosed approaches allow for independent modeling by multiple entities, each using their own datasets and features, thus enhancing the scalability of the predictive modeling.

The disclosed approaches provide model agnosticism, as they are not tied to any specific type of predictive model, thereby allowing the use of various machine learning algorithms. This flexibility ensures that these approaches can be adapted to different technical requirements and advancements in machine learning technologies.

In making use of residuals to identify and correct prediction errors, the disclosed approaches provide systematic improvement of predictive model accuracy and performance. These technical aspects help to ensure that adjustments to predictive models are data-driven and targeted, which leads to more reliable and robust models.

Thus, in view of the above, the disclosed approaches provide a technical solution to the technical problem of suboptimal predictive model accuracy by introducing methodologies and tools that leverage residuals and interactive adjustments from secondary entities. These approaches enhance model performance, maintain data privacy, and are adaptable to various prediction tasks and machine learning frameworks. By addressing the limitations of conventional predictive models, the disclosed approaches offer a significant advancement in the field of predictive analytics. The systematic and collaborative approach ensures that the models are not only more accurate but also more robust and reliable, thereby providing a technical solution to a pressing technical problem.

Furthermore, the technical solution involves predictive models, e.g., statistical models, machine learning models, and/or other computational techniques, having specific characteristics which produce adjusted predictions representing the enhanced and more accurate forecast or estimation for each data point in the modeling cohort and which, in effect, incorporate the insights from both a primary model and a residual model.

As discussed herein, the technical solution is focused on specific improvements in the accuracy of predictive models. It is the implementation of a specific implementation of multiple predictive models, not the mere use of a computer, that improves the existing technological process by providing more accurate predictive models without requiring a significant exchange of data. Thus, the technical solution provides a specific approach that improves the relevant technology, as opposed to being directed to an abstract result or effect which merely invokes generic processes and machinery.

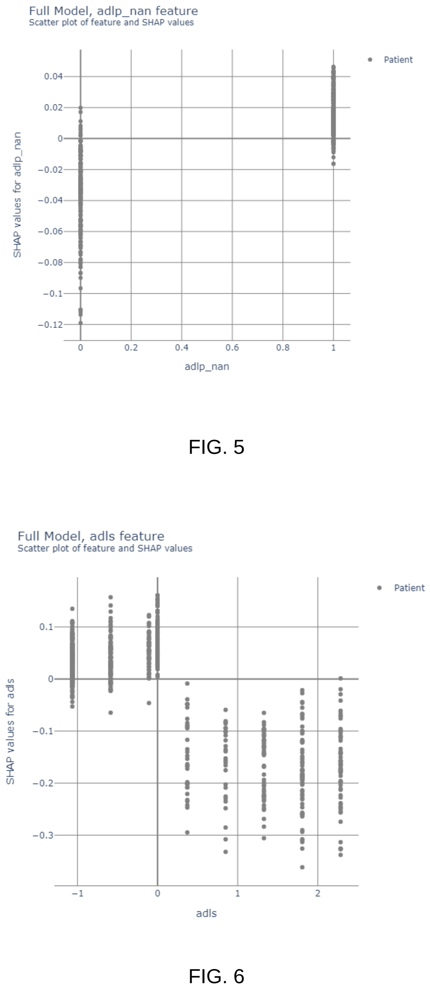

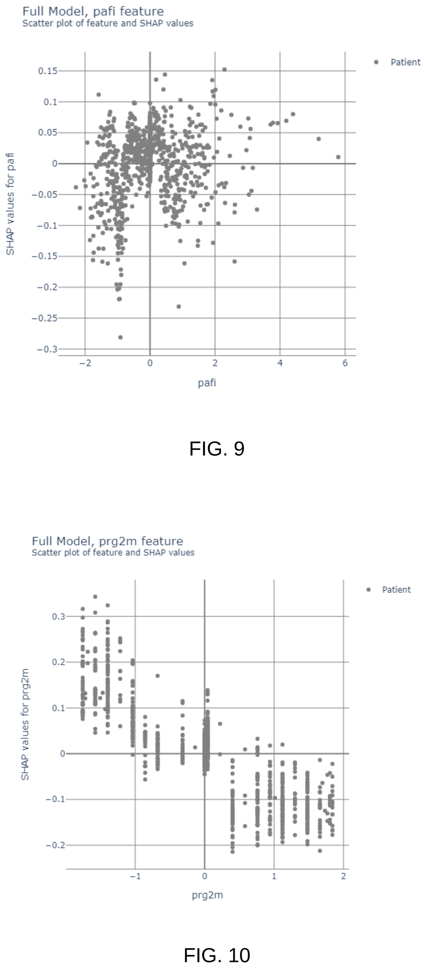

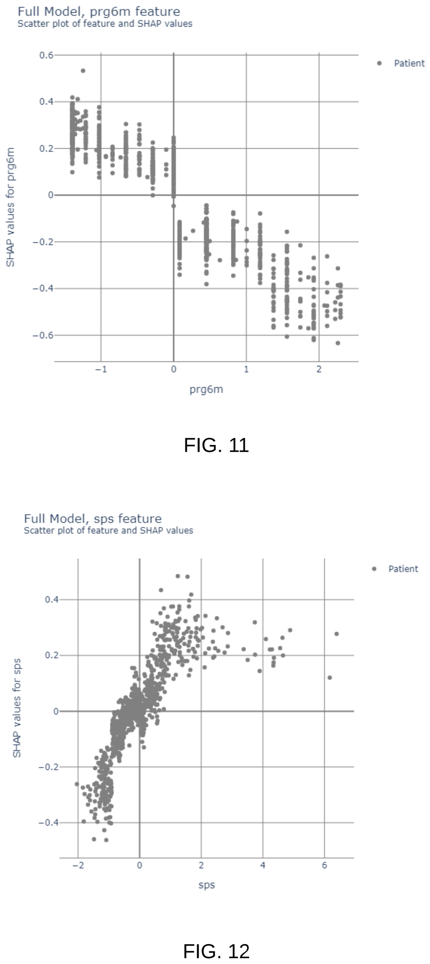

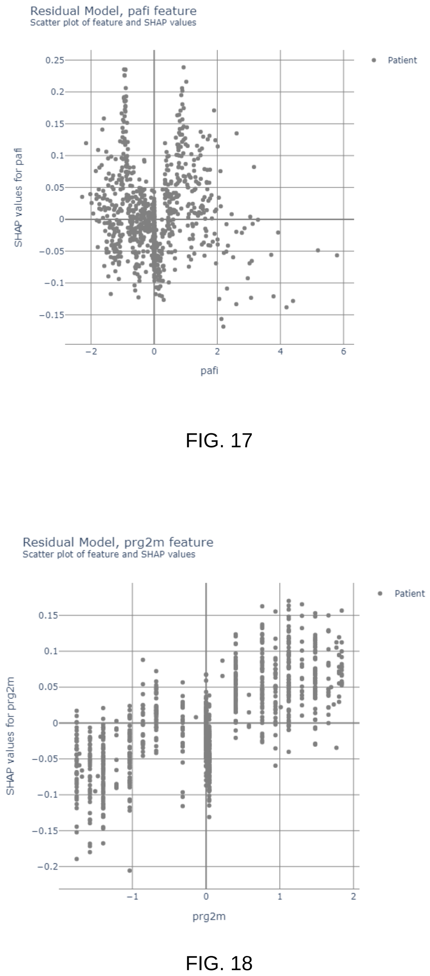

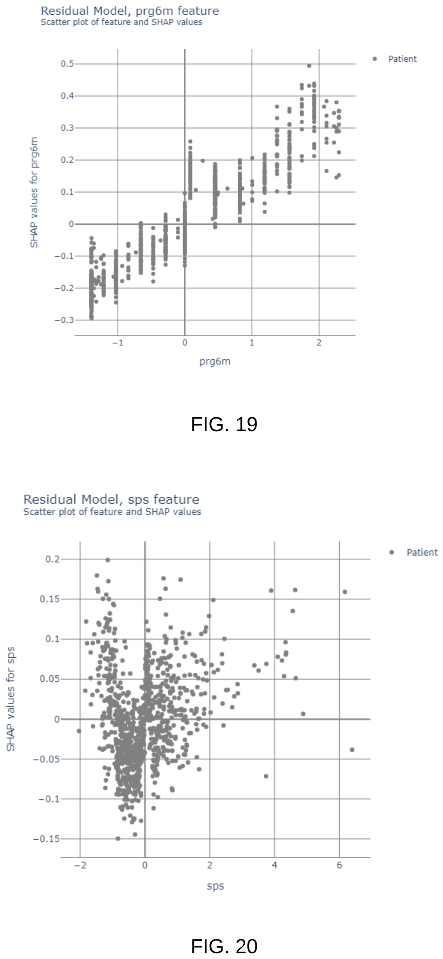

Figures 5-22 relate to an example of the disclosed approaches applied to a publicly-available dataset for a cohort of patients. The dataset is a cohort of 9,105 critically ill patients from 1989-1994 (Vanderbilt University Department of Biostatistics, Professor Frank Harrell 2022). Each row (or record) is a hospitalized patient record meeting the criteria for nine disease categories. The prediction task was an ordinal regression to predict the functional disability of the patient on a five-point scale. The dataset included 35 features related to the patients' demographics and health.

To demonstrate the approaches described herein, three models were trained using different feature sets. The 35 features available in the dataset were split between a Primary Model (17 features) and a Residual Model (18 features). This split of features was meant to illustrate that the Primary Model and the Residual Model are independent of one another, i.e., they need not (and typically would not) use the same feature sets. A third, "Full Model," was trained with all 35 features to represent an ideal scenario in which all available information is shared freely to make the best possible prediction. In this example, Xgboost regression was used to train the Primary Model and the Residual Model on 80% of the dataset (i.e., the training set), with minimal hyperparameter tuning, and the models were evaluated on a 20% holdout test set (i.e., the testing set).

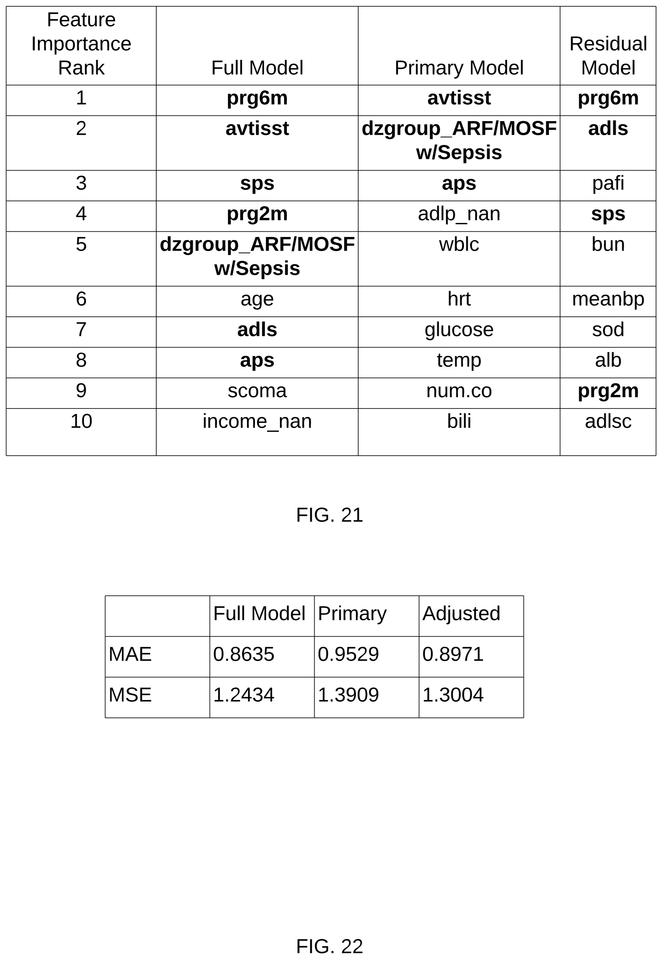

Figure 22 is a table showing the test set error for three sets of outputs (or predictions): the outputs of the Primary Model (e.g., the client's model) without any adjustments, the outputs of an "Adjusted Model" corresponding to the outputs of the Primary Model adjusted by the outputs of the Residual Model, in accordance with the approaches described herein, and the outputs of the Full Model. The three sets of outputs were evaluated on mean absolute error (MAE) and mean squared error (MSE) on the withheld test set. As expected, the Full Model, trained on all features, performed best. Notably, the Adjusted Model (i.e., the Primary Model outputs adjusted according to disclosed embodiments) outperformed the unadjusted Primary Model.

Figures 5-20 are scatter plots of selected features of the three models in the example (i.e., Full, Primary, and Residual) and their Shapley Additive Explanations (SHAP). SHAP is a game theory-based method for explaining the output of machine learning models. SHAP can be used to calculate a value for each feature that represents its contribution to the output of a model. SHAP values show how each feature affects each final prediction, the significance of each feature compared to others, and the model's reliance on the interaction between features. Various other evaluation and/or explanation techniques may be used, e.g., Local Interpretable Model-agnostic Explanations (LIME).

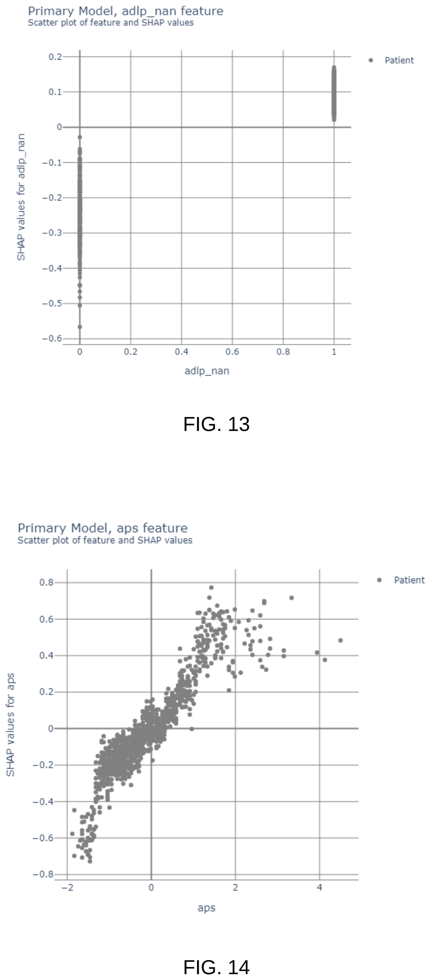

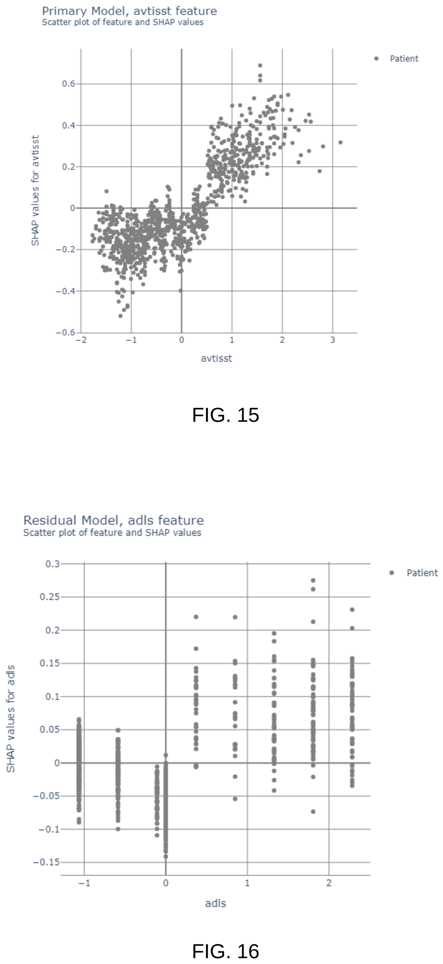

Figures 5-12 are scatter plots of selected features used in the Full Model and their SHAP values. Similarly, Figures 13-15 are plots of selected features in the Primary Model, and Figures 16-20 are plots of selected features in the Residual Model. Each dot in the plots represents a patient in the cohort.

The y-axis of the plots represents the SHAP values for a particular feature/patient, which is the magnitude (or impact) of the feature on the model predictions. Positive y-axis values correspond to relatively higher SHAP values and therefore higher predicted values, and negative y-axis values corresponding to relatively lower SHAP values and therefore lower predicted values. Points near the top and bottom of the plot indicate a higher impact on the model prediction.

The x-axis of the plots represents the standardized values of the particular feature/patient. The points on the left side of the plot (i.e., the negative range of the x-axis) indicate that the patient has a relatively lower value for the particular feature, whereas points on the right side of the plot (i.e., the positive range of the x-axis) indicate that the patient has a relatively higher value for the particular feature.

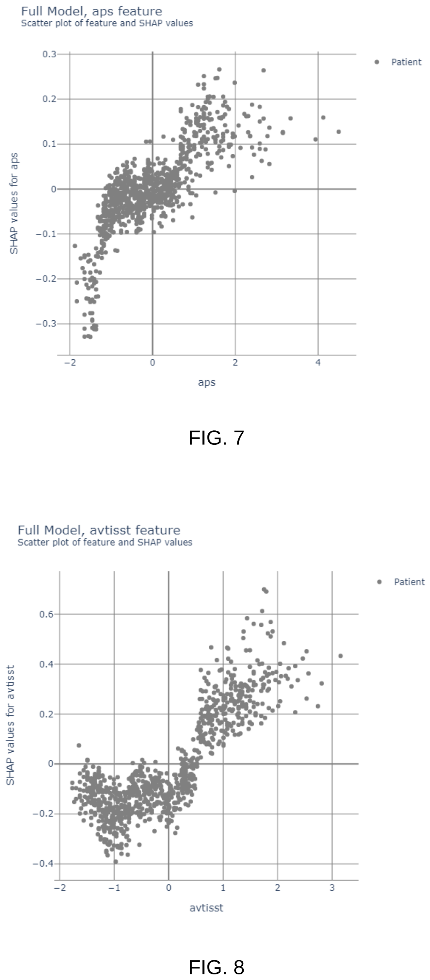

The plots provide a qualitative sense of the consistency in the impact of particular features between the Full Model and the Primary and Residual Models. For example, as shown in Fig. 8, feature *avtisst* has a high density of points in the negative direction of SHAP values (i.e., negative y-axis values) and in the lower values for the patient feature (i.e., negative x-axis values) in the Full Model. The feature also has a high density of points in the higher range of SHAP values (i.e., positive y-axis values) and in the positive values for the patient feature (i.e., positive x-axis values) in the Full Model. These characteristics are consistent with the impact of the feature in the Primary Model, as shown in Fig. 15, indicating that the predictive power, e.g., SHAP values (represented on the y-axis), and patient feature values (represented on the x-axis) of the feature are stable across different models.

In contrast, the directionality of feature impact on model output is generally not consistent between the Full Model and the Residual Model. This is because the residual prediction is expected to be an adjustment to the primary prediction, i.e., in essence a correction to overestimation or underestimation by the Primary Model, because the Residual Model is designed to contribute additional data and/or features which may not have been available to the Full Model (or Primary Model). For example, feature *adls* in essence changes direction, such that relatively high values, e.g., SHAP values (positive y-axis), have a positive feature impact on the patients with relatively lower feature values (negative x-axis) in the Full Model (see Fig. 6), but a negative feature impact (negative y-axis) in the Residual Model (see Fig. 16).

Figure 21 is a table of feature importance rank, based on SHAP values for the Full Model, Primary Model, and Residual Model. The rankings compare the usefulness of features in the Full Model, Primary Model, and Residual Model. Specifically, the table helps in understanding whether a feature that is important in the Full Model remains significant in the Primary and Residual Models and, consequently, the Adjusted Model. For example, the feature *avtisst* is highly important in the Full Model, ranking as the second most important feature. In the Primary Model, it still ranks high, coming in as the most important feature, indicating its consistent significance.

The order of feature importance is consistent between the Full Model and the Primary Model, as expected. For example, *avtisst* feature is second in feature importance for the Full Model and first for the Primary Model. It should be noted that the Full Model features *prg6m* and *sps* were assigned to the Residual Model, so they do not appear in the feature importance ranking for the Primary Model.

The order of feature importance is also consistent between the Full Model and the Residual Model, as expected. For example, the feature *prg6m* is first in feature importance for the Full Model and first in feature importance for the Residual Model. The feature sps is third in feature importance for the Full Model and fourth in feature importance for the Residual Model.

Aspects of the presently taught approaches may be embodied in the form of a system, a computer program product, or a method. Similarly, aspects of the presently taught approaches may be embodied as hardware, software, or a combination of both. Aspects of the presently taught approaches may be embodied as a computer program product saved on and/or conveyed by one or more computer-readable media in the form of computer-readable program code embodied thereon.

The computer-readable medium may be a computer-readable storage medium. A computer-readable storage medium may be, for example, an electronic, optical, magnetic, electromagnetic, infrared, or semiconductor system, apparatus, or device, or any combination thereof. In some examples a computer-readable storage medium may be termed "non-transitory". The computer-readable medium may also or alternatively be a transmission medium by which instructions may be conveyed. A computer-readable transmission medium may include carrier waves, transmission signals or the like. A computer-readable transmission medium may convey instructions between components of a single computer system and/or between plural separate computer systems.

Computer program code in embodiments of the presently taught approaches may be written in any suitable programming language. The program code may execute on a single computer, or on a plurality of computers. The computer may include a processing unit in communication with a computer-usable medium, where the computer-usable medium contains a set of instructions, and where the processing unit is designed to carry out the set of instructions.

Therefore, from one perspective, there have been described approaches for improving accuracy of a primary predictive model based on a residual model. A method includes determining, using a primary predictive model, a first set of training prediction residuals based on a first labeled training set and a first set of testing prediction residuals based on a first labeled testing set. A second dataset includes a second labeled training set, labeled based on the first set of training prediction residuals, and a second labeled testing set, labeled based on the first set of testing prediction residuals. The method further includes training the residual model using the second labeled training set. The primary predictive model is adjusted based on a set of training predictions and a set of testing predictions of the residual model to produce more accurate predictions.

Further examples of feature combinations taught by the present disclosure are set out in the following numbered clauses.

Clause 1. A method for improving accuracy of a primary predictive model based on a residual model, the method comprising: determining, using a primary predictive model, a first set of training prediction residuals based on a first labeled training set and a first set of testing prediction residuals based on a first labeled testing set; producing a second dataset for a residual model, the second dataset comprising a second labeled training set, labeled based on the first set of training prediction residuals, and a second labeled testing set, labeled based on the first set of testing prediction residuals; training the residual model using the second labeled training set; determining, using the residual model, a set of training predictions based on the second labeled training set and a set of testing predictions based on the second labeled testing set; and adjusting the primary predictive model based on the set of training predictions and the set of testing predictions of the residual model to produce more accurate predictions.

Clause 2. The method of clause 1, further comprising: producing a first dataset for the primary predictive model, the first dataset comprising the first labeled training set and the first labeled testing set; and training the primary predictive model using the first labeled training set.

Clause 3. The method of clause 2, wherein said producing the first dataset for the primary predictive model is based on a first set of historical patient-level data and said producing the second dataset for the residual model is based on a second set of historical patient-level data, the first set of patient-level data being different from the second set of patient-level data.

Clause 4. The method of clause 3, wherein said producing the second dataset for the residual model comprises receiving values and labels of the first set of training prediction residuals and values and labels of the first set of testing prediction residuals, without receiving data from the first set of historical patient-level data.

Clause 5. The method of any preceding clause, wherein said producing the first dataset for the primary predictive model comprises defining a modeling cohort and a prediction task.

Clause 6. The method of clause 5, wherein the prediction task comprises predicting clinical trial enrollment.

Clause 7. The method of clause 5 or 6, wherein the prediction task comprises predicting functional disability of a patient.

Clause 8. The method of any preceding clause, wherein said determining the first set of training prediction residuals and the first set of testing prediction residuals comprises: performing predictions based on the first labeled training set and the first labeled testing set; and comparing the predictions to labels of the first labeled training set and the first labeled testing set to compute respective residuals thereof.

Clause 9. The method of any preceding clause, wherein said adjusting the primary predictive model comprises subtracting the set of training predictions and the set of testing predictions of the residual model from predictions of the primary predictive model, the predictions of the primary predictive model being based on the first labeled training set and the first labeled testing set.

Clause 10. The method of clause 9, wherein said adjusting the primary predictive model comprises retuning hyperparameters of the primary predictive model to cause a subsequent set of training prediction residuals and a subsequent set of testing prediction residuals to be closer to the first set of training labels and the first set of testing labels.

Clause 11. A system for generating a synthetic dataset from road vehicle positioning data, comprising one or more processors in communication with a memory, the memory storing instructions executable by said one or more processors to perform: determining, using a primary predictive model, a first set of training prediction residuals based on a first labeled training set and a first set of testing prediction residuals based on a first labeled testing set; producing a second dataset for a residual model, the second dataset comprising a second labeled training set, labeled based on the first set of training prediction residuals, and a second labeled testing set, labeled based on the first set of testing prediction residuals; training the residual model using the second labeled training set; determining, using the residual model, a set of training predictions based on the second labeled training set and a set of testing predictions based on the second labeled testing set; and adjusting the primary predictive model based on the set of training predictions and the set of testing predictions of the residual model to produce more accurate predictions.

Clause 12. The system of clause 11, the memory further storing instructions executable by said one or more processors to perform: producing a first dataset for the primary predictive model, the first dataset comprising the first labeled training set and the first labeled testing set; and training the primary predictive model using the first labeled training set.

Clause 13. The system of clause 12, wherein said producing the first dataset for the primary predictive model is based on a first set of historical patient-level data and said producing the second dataset for the residual model is based on a second set of historical patient-level data, the first set of patient-level data being different from the second set of patient-level data.

Clause 14. The system of clause 11, 12 or 13, wherein said adjusting the primary predictive model comprises subtracting the set of training predictions and the set of testing predictions of the residual model from predictions of the primary predictive model, the predictions of the primary predictive model being based on the first labeled training set and the first labeled testing set.

Clause 15. The system of any of clauses 11 to 14, wherein said adjusting the primary predictive model comprises retuning hyperparameters of the primary predictive model to cause a subsequent set of training prediction residuals and a subsequent set of testing prediction residuals to be closer to the first set of training labels and the first set of testing labels.

Clause 16. A computer-readable medium comprising instructions that, when executed by one or more processors of a computer, cause said one or more processors to become configured to perform: determining, using a primary predictive model, a first set of training prediction residuals based on a first labeled training set and a first set of testing prediction residuals based on a first labeled testing set; producing a second dataset for a residual model, the second dataset comprising a second labeled training set, labeled based on the first set of training prediction residuals, and a second labeled testing set, labeled based on the first set of testing prediction residuals; training the residual model using the second labeled training set; determining, using the residual model, a set of training predictions based on the second labeled training set and a set of testing predictions based on the second labeled testing set; and adjusting the primary predictive model based on the set of training predictions and the set of testing predictions of the residual model to produce more accurate predictions.

Clause 17. The computer-readable medium of clause 16, further comprising instructions that, when executed by one or more processors of a computer, cause said one or more processors to become configured to perform: producing a first dataset for the primary predictive model, the first dataset comprising the first labeled training set and the first labeled testing set; and training the primary predictive model using the first labeled training set.

Clause 18. The computer-readable medium of clause 17, wherein said producing the first dataset for the primary predictive model is based on a first set of historical patient-level data and said producing the second dataset for the residual model is based on a second set of historical patient-level data, the first set of patient-level data being different from the second set of patient-level data.

Clause 19. The computer-readable medium of clause 16, 17 or 18, wherein said adjusting the primary predictive model comprises subtracting the set of training predictions and the set of testing predictions of the residual model from predictions of the primary predictive model, the predictions of the primary predictive model being based on the first labeled training set and the first labeled testing set.

Clause 20. The computer-readable medium of any of clauses 16 to 19, wherein said adjusting the primary predictive model comprises retuning hyperparameters of the primary predictive model to cause a subsequent set of training prediction residuals and a subsequent set of testing prediction residuals to be closer to the first set of training labels and the first set of testing labels.

The above discussion is meant to be illustrative of the principles and various embodiments of the presently taught approaches. Numerous variations and modifications will become apparent to those skilled in the art once the above disclosure is fully appreciated. It is intended that the following claims be interpreted to embrace all such variations and modifications.

## Claims

1. A method for improving accuracy of a primary predictive model based on a residual model, the method comprising:
determining, using a primary predictive model, a first set of training prediction residuals based on a first labeled training set and a first set of testing prediction residuals based on a first labeled testing set;
producing a second dataset for a residual model, the second dataset comprising a second labeled training set, labeled based on the first set of training prediction residuals, and a second labeled testing set, labeled based on the first set of testing prediction residuals;
training the residual model using the second labeled training set;
determining, using the residual model, a set of training predictions based on the second labeled training set and a set of testing predictions based on the second labeled testing set; and
adjusting the primary predictive model based on the set of training predictions and the set of testing predictions of the residual model to produce more accurate predictions.

2. The method of claim 1, further comprising:
producing a first dataset for the primary predictive model, the first dataset comprising the first labeled training set and the first labeled testing set; and
training the primary predictive model using the first labeled training set.

3. The method of claim 2, wherein said producing the first dataset for the primary predictive model is based on a first set of historical patient-level data and said producing the second dataset for the residual model is based on a second set of historical patient-level data, the first set of patient-level data being different from the second set of patient-level data.

4. The method of claim 3, wherein said producing the second dataset for the residual model comprises receiving values and labels of the first set of training prediction residuals and values and labels of the first set of testing prediction residuals, without receiving data from the first set of historical patient-level data.

5. The method of any preceding claim, wherein said producing the first dataset for the primary predictive model comprises defining a modeling cohort and a prediction task.

6. The method of claim 5, wherein the prediction task comprises predicting clinical trial enrollment.

7. The method of claim 5, wherein the prediction task comprises predicting functional disability of a patient.

8. The method of any preceding claim, wherein said determining the first set of training prediction residuals and the first set of testing prediction residuals comprises:
performing predictions based on the first labeled training set and the first labeled testing set; and
comparing the predictions to labels of the first labeled training set and the first labeled testing set to compute respective residuals thereof.

9. The method of any preceding claim, wherein said adjusting the primary predictive model comprises subtracting the set of training predictions and the set of testing predictions of the residual model from predictions of the primary predictive model, the predictions of the primary predictive model being based on the first labeled training set and the first labeled testing set.

10. The method of claim 9, wherein said adjusting the primary predictive model comprises retuning hyperparameters of the primary predictive model to cause a subsequent set of training prediction residuals and a subsequent set of testing prediction residuals to be closer to the first set of training labels and the first set of testing labels.

11. A system for improving accuracy of a primary predictive model based on a residual model, the system comprising:
one or more processors in communication with a memory, the memory storing instructions executable by said one or more processors to perform:
determining, using a primary predictive model, a first set of training prediction residuals based on a first labeled training set and a first set of testing prediction residuals based on a first labeled testing set;
producing a second dataset for a residual model, the second dataset comprising a second labeled training set, labeled based on the first set of training prediction residuals, and a second labeled testing set, labeled based on the first set of testing prediction residuals;
training the residual model using the second labeled training set;
determining, using the residual model, a set of training predictions based on the second labeled training set and a set of testing predictions based on the second labeled testing set; and
adjusting the primary predictive model based on the set of training predictions and the set of testing predictions of the residual model to produce more accurate predictions.

12. The system of claim 11, the memory further storing instructions executable by said one or more processors to perform:
producing a first dataset for the primary predictive model, the first dataset comprising the first labeled training set and the first labeled testing set; and
training the primary predictive model using the first labeled training set.

13. The system of claim 11 or 12, wherein said adjusting the primary predictive model comprises subtracting the set of training predictions and the set of testing predictions of the residual model from predictions of the primary predictive model, the predictions of the primary predictive model being based on the first labeled training set and the first labeled testing set.

14. The system of any of claims 11 to 13, wherein said adjusting the primary predictive model comprises retuning hyperparameters of the primary predictive model to cause a subsequent set of training prediction residuals and a subsequent set of testing prediction residuals to be closer to the first set of training labels and the first set of testing labels.

15. A computer-readable medium comprising instructions that, when executed by one or more processors of a computer, cause said one or more processors to become configured to perform the method of any of claims 1 to 10.
